# EUROPEAN PATENT SPECIFICATION

(11) **EP 3 128 990 B1**
(45) Date of publication and mention of the grant of the patent: **23.08.2017**
(21) Application number: 15712651.7
(22) Date of filing: 30.03.2015
(51) Int. Cl.: A61K 8/27, A61K 8/34, A61Q 5/06

(54) **METHOD OF SHAPING HAIR**
HAARFORMUNGSVERFAHREN
PROCÉDÉ DE MISE EN FORME DES CHEVEUX

(30) Priority: 10.04.2014 EP 14164173
(43) Date of publication of application: 15.02.2017
(73) Proprietor: Unilever PLC, London, Greater London EC4Y 0DY (GB); Unilever NV, 3013 AL Rotterdam (NL)
(72) Inventor: PAUL, Prem, Kumar, Cheyalazhagan, Wirral Merseyside CH63 3JW (GB); PYE, Susan, Wirral Merseyside CH63 3JW (GB)
(74) Representative: James, Helen Sarah
(86) International application number: PCT/EP2015/056866
(87) International publication number: WO 2015/155048

(56) References cited:
- US-A- 5 553 630
- US-A1- 2005 238 600
- US-A1- 2011 223 124

## Description

### Field of the Invention

This invention relates to a method of shaping hair, and more particularly a method of shaping hair without breaking the hair disulfide bonds.

### Background and Prior Art

Many people with naturally kinky, curly, or even wavy hair often desire to straighten their hair. Permanent hair straightening compositions that are on the market are based on chemical treatment of the hair in a two-step process using reducing agents to break hair disulfide bonds, followed by a neutralisation or oxidation step to re-establish new disulfide bonds in the desired configuration. Such systems have various negatives associated with them; in that the process itself is difficult to conduct, in many instances this straightening process is undertaken by a qualified hairdresser in a professional salon. Furthermore the straightening process damages the hair, has an unpleasant odour and can cause irritation to the scalp.

US patent US 2005/238600 discloses a method of shaping the hair comprising 1) applying a zinc oxide hair lotion to the hair, 2) applying waving lotion so as to break the disulfide bond, 3) applying a neutralizer, 4) styling the hair.

Surprisingly we have found that hair can be shaped without causing the chemical damage which is traditionally associated with permanent hair straightening processes involving breakage of the hair disulfide bonds. Advantageously the method of the invention does not necessarily require the use of high temperature heated implements such as straightening irons and can be accomplished by a consumer without intervention of a professional hairdresser. Furthermore, hair shaped with the method of the invention remains shaped even after subsequent washing. The method of the invention is also particularly suitable for damaged hair such as oxidatively treated (e.g. bleached) hair.

### Summary of the Invention

The present invention provides a method of shaping hair comprising the following sequential steps:
(i) topically applying to dry hair a hair treatmentcomposition comprising from 0.1 to 25wt% dispersed zinc oxide (by weight based on the total weight of the hair treatment composition);
(ii) mechanically shaping the treated hair;
(iii) drying the shaped hair;
(iv) washing the dried hair at least once, and
(v) mechanically re-shaping the washed hair.

### Detailed Description and Preferred Embodiments

Preferably the level of dispersed zinc oxide in the hair treatment composition for use in step (i) of the method of the invention ranges from 0.5 to 5wt% and more preferably from 1 to 3wt% (by weight based on the total weight of the hair treatment composition).

A fully formulated hair treatment composition for use in step (i) of the method of the invention may include further optional ingredients (in addition to the dispersed zinc oxide) to enhance performance and/or consumer acceptability.

For example, a hair treatment composition for use in step (i) of the method of the invention may include a conditioning gel phase, which may be generally characterized as a gel (L•) surfactant mesophase consisting of surfactant bilayers. Such a conditioning gel phase may be formed from a cationic surfactant, a high melting point fatty alcohol and an aqueous carrier. Typically these components are heated to form a mixture, which is cooled under shear to room temperature. The mixture undergoes a number of phase transitions during cooling, normally resulting in a gel (L.) surfactant mesophase consisting of surfactant bilayers.

Examples of suitable cationic surfactants which are useful for forming the conditioning gel phase include quaternary ammonium cationic surfactants corresponding to the following general formula:

[N(R¹)(R²)(R³)(R⁴)]⁺ (X)⁻

in which R¹, R², R³, and R⁴ are each independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halide, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of 12 carbons, or higher, can be saturated or unsaturated.

Specific examples of such quaternary ammonium cationic surfactants of the above general formula are cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, dipalmitoylethyldimethylammonium chloride, PEG-2 oleylammonium chloride and salts of these, where the chloride is replaced by other halide (e.g., bromide), acetate, citrate, lactate, glycolate, phosphate nitrate, sulphate, or alkylsulphate.

In a preferred class of cationic surfactant of the above general formula, R¹ is a C₁₆ to C₂₂ saturated or unsaturated, preferably saturated, alkyl chain and R², R³ and R⁴ are each independently selected from CH₃ and CH₂CH₂OH, preferably CH₃.

Specific examples of such preferred quaternary ammonium cationic surfactants for use in forming the conditioning gel phase are cetyltrimethylammonium chloride (CTAC), behenyltrimethylammonium chloride (BTAC) and mixtures thereof.

Mixtures of any of the above-described cationic surfactants may also be suitable.

The level of cationic surfactant suitably ranges from 0.1 to 10 wt%, preferably from 0.2 to 5 wt% and more preferably from 0.25 to 4 wt% (by total weight of cationic surfactant based on the total weight of the hair treatment composition).

By "high melting point" in the context of this invention is generally meant a melting point of 25°C or higher. Generally the melting point ranges from 25°C up to 90°C, preferably from 40°C up to 70° C and more preferably from 50°C up to 65°C.

The high melting point fatty alcohol can be used as a single compound or as a blend or mixture of at least two high melting point fatty alcohols. When a blend or mixture of fatty alcohols is used, the melting point means the melting point of the blend or mixture.

Suitable fatty alcohols of this type have the general formula R-OH, where R is an aliphatic carbon chain. Preferably R is a saturated aliphatic carbon chain comprising from 8 to 30 carbon atoms, more preferably from 14 to 30 carbon atoms and most preferably from 16 to 22 carbon atoms.

R can contain, in addition to carbon and hydrogen atoms, ether linkages, and other groups such as amino groups.

Most preferably, the fatty alcohol has the general formula CH₃(CH₂)ₙ OH, where n is an integer from 7 to 29, preferably from 15 to 21.

Specific examples of suitable fatty alcohols are cetyl alcohol, stearyl alcohol, behenyl alcohol, and mixtures thereof. Cetyl alcohol, stearyl alcohol and mixtures thereof are particularly preferred.

Mixtures of any of the above-described fatty alcohols may also be suitable.

The level of fatty alcohol suitably ranges from 0.01 to 10 wt%, preferably from 0.1 to 8 wt%, more preferably from 0.2 to 7 wt% and most preferably from 0.3 to 6 wt% (by weight based on the total weight of the hair treatment composition).

The weight ratio of cationic surfactant to fatty alcohol is suitably from 1:1 to 1:10, preferably from 1:1.5 to 1:8, optimally from 1:2 to 1:5.

Advantageously, the method of the invention does not require the breakage of hair disulfide bonds, and hair treatment compositions for use in the invention do not require the incorporation of reducing agents. It is preferred that such materials, if included at all, are present in minor quantities only.

The term "reducing agent" in the context of this invention means an agent which is effective to break hair disulfide bonds when applied to hair for a period ranging from 3 to 15 minutes and at a temperature ranging from 20 to 30ºC. Examples of such reducing agents are ammonium thioglycolate (in a solution having a pH of between 7 and 10.5), glyceryl monothioglycolate (employed at a pH of less than 7), thioglycolic acid, dithioglycolic acid, mercaptoethyl amine, mercaptopropionic acid, dithioglycolate and alkali metal or ammonium sulfites or bisulfites.

A hair treatment composition for use in step (i) of the method of the invention will preferably include from 0 to 0.1 wt%, more preferably from 0 to 0.01 wt%, and most preferably from 0 to 0.001 wt% reducing agents as defined above (by weight based on the total weight of the hair treatment composition).

Hair treatment compositions for use in step (i) of the method of the invention will generally comprise at least 60 wt%, preferably at least 70 wt% and more preferably at least 80 wt% water (by weight based on the total weight of the hair treatment composition). Preferably, the hair treatment composition comprises no more than 95 wt% and more preferably no more than 90 wt% water (by weight based on the total weight of the hair treatment composition).

Other organic solvents may also be present, such as lower alkyl alcohols and polyhydric alcohols. Examples of lower alkyl alcohols include C₁ to C₆ monohydric alcohols such as ethanol and isopropanol. Examples of polyhydric alcohols include propylene glycol, hexylene glycol, glycerin, and propanediol. Mixtures of any of the above described organic solvents may also be used.

The hair treatment composition may also incorporate other optional ingredients to enhance performance and/or consumer acceptability. Suitable optional ingredients include: preservatives, colouring agents, chelating agents, antioxidants, fragrances, antimicrobials, antidandruff agents, cationic conditioning polymers, styling ingredients, sunscreens, proteins and hydrolysed proteins.

Preferably, the hair treatment composition is a single dose composition. The term "single dose" in the context of this invention means that the hair treatment composition is to be applied to the hair in one go.

Preferably, the hair treatment composition is applied to the hair in the form of a 100 to 300 ml single dose, more preferably a 150 to 250 ml single dose.

Preferably, the hair treatment composition is applied to the hair at a temperature from 15 to 40ºC, and more preferably at a temperature from 20 to 30ºC.

In step (i) of the method of the invention, a hair treatment composition as described above is applied to dry hair. The term "dry hair" in the context of this invention generally means hair from which free water (i.e. water disposed as a film or droplets on the cuticle surface) has been substantially removed. Hair may be dried by exposure to air, by use of a heated hair drying appliance, by rubbing with a water-absorbent article, or by a combination of any of these methods. Preferably, the dry hair will not have been washed or actively wetted, (such as by shampooing, conditioning, rinsing or otherwise treating with an aqueous composition) in the preceding 2 hours and more preferably in the preceding 3 hours prior to topical application of the hair treatment composition in accordance with step (i) of the method of the invention, and will have been permitted to acclimatise to atmospheric conditions. In such circumstances there is substantially no free water present which interferes with the adsorption of the hair treatment composition on application. A suitable indicator of dry hair in the context of this invention would be a hair fibre whose calculated water content does not exceed 25 wt% by weight based on the total weight of the hair fibre.

Preferably, the hair treatment composition is worked through the hair after topical application.

Preferably, after working through the hair, the hair treatment composition is then left to penetrate the hair for a period of at least 5 up to 90 minutes, more preferably for at least 15 up to 60 minutes and most preferably for at least 20 up to 40 minutes.

At the end of the treatment period, and before the commencement of step (ii) of the method of the invention, the hair treatment composition may optionally be rinsed from the hair (in a so-called "rinse-off" treatment method), but is preferably left on the hair without rinsing (in a so-called "leave-on" treatment method).

Preferably, at the end of the treatment period, and before the commencement of step (ii) of the method of the invention, the hair is dried and the dry hair is then treated with a second hair treatment composition comprising from 0.01 to 5wt% (by weight based on the total weight of the second hair treatment composition) fatty acid.

Suitable fatty acids in the context of this invention include aliphatic carboxylic acids of formula RCOOH, where R is a linear or branched alkyl or alkenyl chain containing from 12 to 22 carbon atoms and 0, 1, 2 or 3 double bond(s).

Specific examples of such materials include lauric acid, myristic acid, palmitic acid, stearic acid, arachic acid, behenic acid, dimethyloctanoic acid, butylheptylnonanoic acid, hexenoic acid, octenoic acid, decenoic acid, dodecenoic acid, tetradecenoic acid, hexadecenoic acid, octadecenoic acid, eicosenoic acid, docosenoic acid, linoleic acid and linolenic acid.

Preferred fatty acids for use in the invention include those of the above formula in which R is a linear alkyl or alkenyl chain containing from 16 to 18 carbon atoms and 0 or 1 double bond(s). Specific examples of such materials include palmitic acid, palmitoleic acid, stearic acid and oleic acid. Oleic acid is particularly preferred.

Preferably the level of fatty acid ranges from 0.05 to 2wt% and more preferably from 0.1 to 0.2wt%, by weight based on the total weight of the second hair treatment composition.

A fully formulated second hair treatment composition for use in the method of the invention may also include further optional ingredients; as are described above in relation to the zinc oxide-containing composition.

In particular, a second hair treatment composition for use in the method of the invention will preferably include from 0 to 0.1 wt%, more preferably from 0 to 0.01 wt%, and most preferably from 0 to 0.001 wt% reducing agents as defined above (by weight based on the total weight of the second hair treatment composition).

Preferably, the second hair treatment composition is topically applied to the hair and worked through the hair as described above in relation to the zinc oxide-containing composition.

Preferably, after working through the hair, the second hair treatment composition is then left to penetrate the hair for a period of at least 5 up to 90 minutes, more preferably for at least 15 up to 60 minutes and most preferably for at least 20 up to 40 minutes.

At the end of the treatment period, and before the commencement of step (ii) of the method of the invention, the second hair treatment composition may optionally be rinsed from the hair (in a so-called "rinse-off" treatment method), but is preferably left on the hair without rinsing (in a so-called "leave-on" treatment method).

In step (ii) of the method of the invention, the treated hair is mechanically shaped. Preferably, the hair is mechanically straightened. For example, the hair may be pulled, combed, smoothed, pressed or flattened into a straightened configuration.

A hot tool, such as an electrically heated flat hair iron or hand-held hair dryer, may be used in the mechanical shaping step. Such tools apply high levels of heat directly to the hair. Most operate in the 45ºC to 250ºC range, and are usually employed at temperature settings ranging from 50°C to 220°C, depending on the particular tool.

However, the present inventors have surprisingly found that the use of hot tools is not essential in the method of the invention. This is especially advantageous for consumers who wish to reduce or avoid the exposure of their hair to high temperatures, for example if their hair is fragile or overprocessed from previous chemical treatments such as bleaching and perming.

Accordingly the mechanical shaping of the hair in step (ii) of the method of the invention is preferably conducted at a temperature from 15 to 40ºC, more preferably at a temperature from 20 to 30ºC.

Most preferably in step (ii) of the method of the invention the hair is mechanically straightened by combing it into a straightened configuration at a temperature from 20 to 30ºC.

Preferably in step (iii) of the method of the invention, the shaped hair is dried by exposure to air.

The present inventors have surprisingly found that hair may be durably shaped by the method of the invention. The term "durably shaped" in the context of this invention means that the hair shape persists after washing.

In step (iv) of the method of the invention, the hair is washed, preferably once or twice, and preferably with a cleansing composition. A cleansing composition for use in step (iv) of the method of the invention (such as a shampoo or shower gel) will suitably have an aqueous continuous phase incorporating at least one anionic surfactant. Preferred anionic surfactants include sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20), and mixtures thereof. The amount of anionic surfactant generally ranges from 5 to 20% by total weight anionic surfactant(s) based on the total weight of the composition.

Preferably in step (v) of the method of the invention, the washed hair is mechanically re-straightened by combing it into a straightened configuration at a temperature from 20 to 30ºC.

The invention is further illustrated with reference to the following, non-limiting Examples.

### EXAMPLES

All ingredients are expressed by weight percent of the total formulation, and as level of active ingredient.

### Example 1

Twice bleached switches of dark brown straight European hair switches were soaked in a 2% dispersion of zinc oxide for 30 minutes. Control switches were soaked in water. All switches were combed straight and were dried in drying cabinets. When dry the switches were washed in base wash (14% SLES solution) combed straight and allowed to dry overnight. Pictures of switches were taken before and after combing. The volumes of the switches were measured using an image analysis rig after combing when dry and at the end of 1 hour of high humidity (30°C and 80%RH). The results are given in Table 1 below.

**Table 1**

| **Treatment** | **after initial combing** | | **after high humidity** | |
|---|---|---|---|---|
| | volume | %benefit | volume | %benefit |
| Control (water) | 8991 | 0 | 10352 | 0 |
| 2% ZnO | 6434 | 28.4 | 7616 | 26.4 |

The results show that the treatment with zinc oxide according to the method of the invention imparts good shape benefits even after 1 wash and after high humidity compared to the control.

### Example 2

Twice bleached switches of dark brown straight European hair switches were soaked in a 2% dispersion of zinc oxide for 30 minutes. When the switches were dry, oleic acid at a dose of 0.1 g per gram of hair was applied on the switches. Twice bleached switches that were soaked in water for 30 minutes and dried and had only oleic acid applied at a dose of 0.1 g per gram of hair were used as control. All switches were combed straight and were left for 30 minutes before being washed twice in base wash (14% SLES solution), combed straight when wet, and left to dry overnight. Pictures of switches were taken before and after combing. The volumes of the switches were measured using an image analysis rig after combing when dry and at the end of 1 hour of high humidity (30°C and 80%RH). The results are given in Table 2 below.

**Table 2**

| **Treatment** | **after initial combing** | | **after high humidity** | |
|---|---|---|---|---|
| | volume | %benefit | volume | %benefit |
| Control (0.1 % oleic acid) | 10718 | 0 | 11471 | 0 |
| 2% ZnO + 0.1 % oleic acid | 8094 | 24.5 | 8924 | 22.2 |

The results show that the treatment with zinc oxide and oleic acid according to the method of the invention imparts good shape benefits even after 2 washes and after high humidity compared to the control.

It was also observed that the switches which were treated according to the method of the invention had superior visual and tactile attributes (such as feel, bounce and flow) compared to those treated with the control.

### Example 3

The switches of Examples 1 and 2 were heat straightened with irons and placed in high humidity chamber for 1 hour. Once again anti-humidity benefits were seen with the switches which were treated according to the method of the invention.

## Claims

1. A method of shaping hair comprising the following sequential steps:
(i) topically applying to dry hair a hair treatmentcomposition comprising from 0.1 to 25wt% dispersed zinc oxide by weight based on the total weight of the hair treatment composition;
(ii) mechanically shaping the treated hair;
(iii) drying the shaped hair;
(iv) washing the dried hair at least once, and
(v) mechanically re-shaping the washed hair.

2. A method according to claim 1, in which the level of dispersed zinc oxide in the hair treatment composition ranges from 0.5 to 5wt% by weight based on the total weight of the hair treatment composition.

3. A method according to claim 1 or 2, in which the hair treatment composition includes from 0 to 0.001wt% reducing agents by weight based on the total weight of the hair treatment composition.

4. A method according to any one of claims 1 to 3, in which the hair is treated at the end of step (i) and before the commencement of step (ii) with a second hair treatment composition comprising from 0.01 to 5wt% by weight based on the total weight of the second hair treatment composition fatty acid.

5. A method according to claim 4, in which the fatty acid is selected from aliphatic carboxylic acids of formula RCOOH formula in which R is a linear alkyl or alkenyl chain containing from 16 to 18 carbon atoms and 0 or 1 double bond(s).

6. A method according to claim 5, in which the fatty acid is oleic acid.

7. A method according to any one of claims 1 to 6, in which in step (ii) the hair is mechanically straightened by combing it into a straightened configuration at a temperature from 20 to 30ºC.

8. A method according to any one of claims 1 to 7, in which in step (iv) the hair is washed once or twice with a cleansing composition.

9. A method according to claim 8, in which the cleansing composition has an aqueous continuous phase incorporating at least one anionic surfactant, and the amount of anionic surfactant ranges from 5 to 20% by total weight anionic surfactant(s) based on the total weight of the composition.

10. A method according to any one of claims 1 to 9, in which the hair to be treated by sequential steps (i) to (v) is oxidatively treated hair.

11. A method according to claim 10, in which the hair to be treated by sequential steps (i) to (v) is bleached hair.

## Patentansprüche

1. Verfahren zur Haarverformung, umfassend die folgenden fortlaufenden Schritte:
(i) topisches Auftragen einer Haarbehandlungszusammensetzung, die 0,1 bis 25 Gew.-% dispergiertes Zinkoxid, in Gewicht, bezogen auf das Gesamtgewicht der Haarbehandlungszusammensetzung, umfasst, auf trockenes Haar,
(ii) mechanisches Formen des behandelten Haars,
(iii) Trocknen des geformten Haars,
(iv) mindestens einmaliges Waschen des getrockneten Haars und
(v) mechanisches Umformen des gewaschenen Haars.

2. Verfahren nach Anspruch 1, in welchem sich die Konzentration des dispergierten Zinkoxids in der Haarbehandlungszusammensetzung von 0,5 bis 5 Gew.-%, in Gewicht, bezogen auf das Gesamtgewicht der Haarbehandlungszusammensetzung, erstreckt.

3. Verfahren nach Anspruch 1 oder 2, in welchem die Haarbehandlungszusammensetzung 0 bis 0,001 Gew.-% Reduktionsmittel, in Gewicht, bezogen auf das Gesamtgewicht der Haarbehandlungszusammensetzung, einbezieht.

4. Verfahren nach irgendeinem der Ansprüche 1 bis 3, in welchem das Haar am Ende des Schritts (i) und vor Beginn des Schritts (ii) mit einer zweiten Haarbehandlungszusammensetzung, die 0,01 bis 5 Gew.-%, in Gewicht, bezogen auf das Gesamtgewicht der zweiten Haarbehandlungszusammensetzung, Fettsäure umfasst, behandelt wird.

5. Verfahren nach Anspruch 4, in welchem die Fettsäure aus aliphatischen Carbonsäuren der Formel RCOOH, worin R eine lineare Alkyl- oder Alkenyl-Kette ist, die 16 bis 18 Kohlenstoffatome und 0 oder 1 Doppelbindung enthält, ausgewählt wird.

6. Verfahren nach Anspruch 5, in welchem die Fettsäure Ölsäure ist.

7. Verfahren nach irgendeinem der Ansprüche 1 bis 6, in welchem das Haar im Schritt (ii) mechanisch geglättet wird, indem es bei einer Temperatur von 20 bis 30°C in eine geglättete Konfiguration zusammengelegt wird.

8. Verfahren nach irgendeinem der Ansprüche 1 bis 7, in welchem das Haar im Schritt (iv) einmal oder zweimal mit einer Reinigungszusammensetzung gewaschen wird.

9. Verfahren nach Anspruch 8, in welchem die Reinigungszusammensetzung eine wässrige kontinuierliche Phase aufweist, die mindestens ein anionisches Tensid einbezieht und wobei sich die Menge des anionischen Tensids von 5 bis 20%, in Gesamtgewicht des (der) anionischen Tensids(e), bezogen auf das Gesamtgewicht der Zusammensetzung, erstreckt.

10. Verfahren nach irgendeinem der Ansprüche 1 bis 9, in welchem das durch die fortlaufenden Schritte (i) bis (v) zu behandelnde Haar oxidativ behandeltes Haar ist.

11. Verfahren nach Anspruch 10, in welchem das durch die fortlaufenden Schritte (i) bis (v) zu behandelnde Haar gebleichtes Haar ist.

## Revendications

1. Procédé de mise en forme des cheveux comprenant les étapes successives suivantes :
(i) application topique aux cheveux secs d'une composition de traitement des cheveux comprenant de 0,1 à 25 % en masse d'oxyde de zinc dispersé en masse rapporté à la masse totale de la composition de traitement des cheveux ;
(ii) mise en forme mécanique des cheveux traités ;
(iii) séchage des cheveux mis en forme ;
(iv) lavage des cheveux séchés au moins une fois, et
(v) re-mise en forme mécanique des cheveux lavés.

2. Procédé selon la revendication 1, dans lequel la teneur en oxyde de zinc dispersé dans la composition de traitement des cheveux est de 0,5 à 5 % en masse rapporté à la masse totale de la composition de traitement des cheveux.

3. Procédé selon la revendication 1 ou 2, dans lequel la composition de traitement des cheveux comprend de 0 à 0,001 % en masse d'agents réducteurs en masse rapporté à la masse totale de la composition de traitement des cheveux.

4. Procédé selon l'une quelconque des revendications 1 à 3, dans lequel les cheveux sont traités à la fin de l'étape (i) et avant le début de l'étape (ii) avec une seconde composition de traitement des cheveux comprenant de 0,01 à 5 % en masse d'acide gras en masse rapporté à la masse totale de la seconde composition de traitement des cheveux.

5. Procédé selon la revendication 4, dans lequel l'acide gras est choisi parmi des acides carboxyliques aliphatiques de formule RCOOH dans laquelle R est une chaîne alkyle ou alcényle linéaire contenant de 16 à 18 atomes de carbone et 0 ou 1 double liaison.

6. Procédé selon la revendication 5, dans lequel l'acide gras est l'acide oléique.

7. Procédé selon l'une quelconque des revendications 1 à 6, dans lequel dans l'étape (ii) les cheveux sont mécaniquement raidis en les peignant dans une configuration raidie à une température de 20 à 30°C.

8. Procédé selon l'une quelconque des revendications 1 à 7, dans lequel dans l'étape (iv) les cheveux sont lavés une fois ou deux fois avec une composition nettoyante.

9. Procédé selon la revendication 8, dans lequel la composition nettoyante présente une phase continue aqueuse incorporant au moins un tensioactif anionique, et la quantité de tensioactif anionique est de 5 à 20 % en masse totale de tensioactif(s) anionique(s) rapporté à la masse totale de la composition.

10. Procédé selon l'une quelconque des revendications 1 à 9, dans lequel les cheveux à traiter par les étapes successives (i) à (v) sont des cheveux traités par oxydation.

11. Procédé selon la revendication 10, dans lequel les cheveux à traiter par les étapes successives (i) à (v) sont des cheveux blanchis.
